# EUROPEAN PATENT APPLICATION

(11) **EP 1 944 096 A1**
(43) Date of publication of application: **16.07.2008**
(21) Application number: 08000590.3
(22) Date of filing: 14.01.2008
(51) Int. Cl.: B06B 1/06, H01L 41/047

(54) **Ultrasonic generator and skin care device using same**

(30) Priority: 15.01.2007 JP 2007006245
(71) Applicant: MATSUSHITA ELECTRIC WORKS, LTD., Kadoma-shi, Osaka (JP)
(72) Inventor: Saida, Itaru, Kadoma-shi Osaka (JP); Nunomura, Mahito, Kadoma-shi Osaka (JP); Matsusaka, Takeshi, Kadoma-shi Osaka (JP)
(74) Representative: Samson & Partner

(57) **Abstract**

An ultrasonic generator includes an ultrasonic vibrator (1) generating ultrasonic vibration and a horn. The horn is of a flat plate-like horn (16) having opposite flat surfaces, one of which forms a connection surface connected to the ultrasonic vibrator and the other of which forms an ultrasonic wave emitting surface (16b) from which ultrasonic waves are emitted toward a target point. A horn-side connection terminal (11) contacts a circumferential portion of the connection surface surrounding the ultrasonic vibrator at a plurality of place thereof to electrically connect to one electrode of the ultrasonic vibrator through flat plate-like horn.

## Description

The present invention relates to an ultrasonic generator in which an ultrasonic vibrator is connected to a horn and a skin care device provided with the ultrasonic generator.

Conventionally, there is known an ultrasonic generator including a horn and an ultrasonic vibrator connected to the horn. The ultrasonic generator is configured to emit an ultrasonic vibration to the outside through the horn by converting an electric vibration generated by an alternating electric field applied thereto into a mechanical vibration.

The ultrasonic generator configured as above is adapted for use in, e.g., a skin care device that, when the horn is pressed against a skin surface, transmits the ultrasonic vibration toward the skin surface or the inside of a living body to thereby provide a desired aesthetic effect (see, e.g., Japanese Patent Laid-open Application No. 2006-181292)).

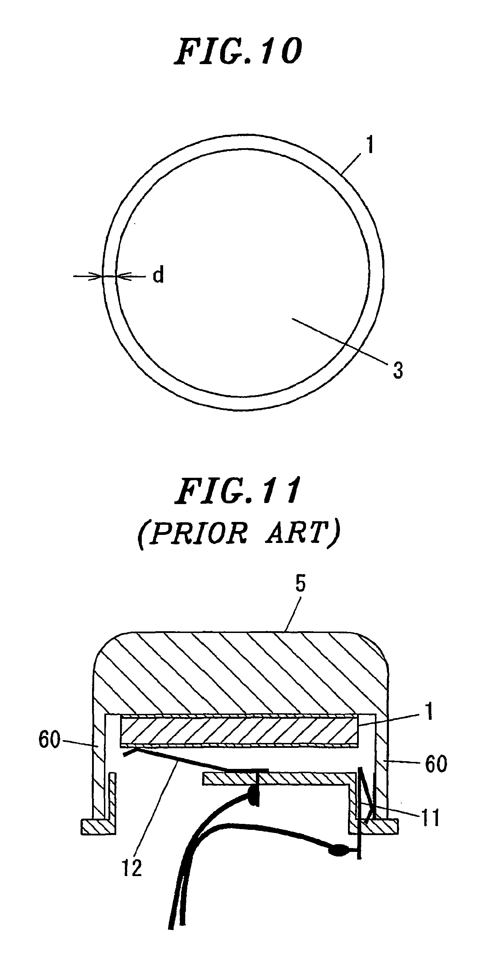

As shown in Fig. 11, in the conventional ultrasonic generator, a bottom-closed tubular horn 5 having a circumferential side wall 60 is used as the horn 5 to which the ultrasonic vibrator 1 is connected. One electrode of the ultrasonic vibrator 1 is electrically connected to a control circuit through the horn 5 by contacting a horn-side connection terminal 11 with the circumferential side wall 60. Further, a vibrator-side connection terminal 12 is directly connected to the other electrode of the ultrasonic vibrator 1. Therefore, both electrodes of the ultrasonic vibrator 1 are electrically connected with the control circuit by the horn-side connection terminal 11 and the vibrator-side connection terminal 12. Such a bottom-closed tubular horn 5 having the circumferential side wall 60 of large area connected with the horn-side connection terminal 11 provides an advantage in that electric power can be reliably supplied to the ultrasonic vibrator 1.

However, the ultrasonic generator provided with the bottom-closed tubular horn 5 suffers from the following problems. Specifically, the ultrasonic wave transmitting horn 5 is usually made of a light metal, e.g., aluminum, or a light alloy. For this reason, it is difficult to produce a complex shape, i.e., a bottom-closed tubular shape, by machining in conformity with strict dimensional requirements for assuring the reliable transmission of ultrasonic vibration. Furthermore, an increased volume of the metal is cut away in the process of producing the horn 5, thereby increasing the production cost. Moreover, the horn 5 becomes large-sized, consequently making the device bulky and heavy.

As a solution to these problems, it would be possible to form the horn 5 of a flat plate shape, in which case however it becomes difficult to reliably supply an electric power to the ultrasonic vibrator 1 through the horn 5.

In view of the problems noted above, the present invention provides an ultrasonic generator in which a horn of flat plate shape is used to thereby make a device employing the same small-sized, lightweight and cost-effective and also to reliably supply an electric power to the ultrasonic vibrator through the horn and a skin care device using the ultrasonic generator.

In accordance with one aspect of the invention, there is provided an ultrasonic generator including: an ultrasonic vibrator generating ultrasonic vibration; and a horn having a connection surface connected to the ultrasonic vibrator and an ultrasonic wave emitting surface from which ultrasonic waves are emitted toward a target point, wherein the horn has a flat plate-like horn having opposite flat surfaces, one of which forms the connection surface and the other of which forms the ultrasonic wave emitting surface, and wherein a horn-side connection terminal contacts a circumferential portion of the connection surface surrounding the ultrasonic vibrator at a plurality of place thereof to electrically connect to one electrode of the ultrasonic vibrator through flat plate-like horn.

With the ultrasonic generator noted above, the task of forming a metallic material into the flat plate-like horn becomes easy and the volume of metal cut away is reduced. This makes it possible to provide the ultrasonic generator in a cost-effective manner. Furthermore, use of the small-sized and lightweight flat plate-like horn is effective in reducing the size and weight of the device as a whole.

Moreover, owing to the fact that the horn-side connection terminal is contacted with the flat plate-like horn by effectively using a circumferential portion of the flat plate-like horn surrounding the ultrasonic vibrator contacted with the connection surface, it is possible to reliably supply the electric power to the ultrasonic vibrator through the flat plate-like horn.

Preferably, the horn-side connection terminal elastically contacts the flat plate-like horn by using a spring structure.

With this configuration, it is possible to prevent a contact pressure of the horn-side connection terminal from being increased excessively, and also possible to manufacture the horn-side connection terminal cost effectively.

It is preferable that the horn-side connection terminal is of a shape conforming to an edge of the ultrasonic vibrator connected to the flat plate-like horn.

The contact between the horn-side connection terminal and the ultrasonic vibrator causes an electric short circuit or damage. With this configuration, however, it is possible to dispose the horn-side connection terminal without contacting the ultrasonic vibrator by effectively using small area.

Further, the horn-side connection terminal includes elastic pieces of an arm shape elastically contacting the flat plate-like horn, the leading end portions of the elastic pieces becoming gradually distanced away from the ultrasonic vibrator as the leading end portions proceed to the leading ends of the elastic pieces and being bent toward the flat plate-like horn.

A leading ends of the elastic pieces may bend toward the ultrasonic vibrator depending on a method of bending the elastic pieces. Since the elastic pieces are formed to be away from the ultrasonic vibrator as it goes to the leading ends thereof which elastically contacts the flat plate-like horn, an electric short circuit or damage due to the contact between the elastic pieces and the ultrasonic vibrator can be avoided.

Furthermore, a vibrator-side connection terminal contacts a circumferential portion of the other electrode of the ultrasonic vibrator surrounded by the vibrator-side connection terminal at a plurality of place thereof for an electric connection.

If a lead wire is soldered to the other electrode of the ultrasonic vibrator, there is a concern of damaging a function of the ultrasonic vibrator or inducing unnecessary heat generation of the ultrasonic vibrator due to a resistance of a solder itself. Since the lead wire is connected to the other electrode through the vibrator-side connection terminal, the connection problem using soldering is solved. The vibrator-side connection terminal is disposed at an inner portion of the horn-side connection terminal so that an electric contact may be made by effectively using small space near the flat plate-like horn.

Preferably, the vibrator-side connection terminal contacts a portion of the ultrasonic vibrator where the ultrasonic vibration is least.

With this configuration, contact failure by the vibration of the ultrasonic vibrator can be avoided.

Further, an insulation member is disposed between the outer horn-side connection terminal and the inner vibrator-side connection terminal.

With this configuration, any electric short circuit by the contact between the horn-side connection terminal and the vibrator-side connection terminal can be avoided.

Furthermore, the ultrasonic generator further including: a base portion mounting the horn-side connection terminal and the vibrator-side connection terminal thereon, each having a spring structure; and a restriction member maintaining a predetermined distance between the base portion and the flat plate-like horn to maintain predetermined contact pressures between the horn-side connection terminal and the flat plate-like horn and between the vibrator-side connection terminal and the ultrasonic vibrator, respectively.

With this configuration, the contact pressures at the horn-side connection terminal and the vibrator-side connection terminal can be respectively maintained at predetermined levels so that reliable supply of an electric power is realized.

It is preferable that the electrode electrically contacting with the vibrator-side connection terminal among both electrodes has a cutout portion d which is formed from the circumferential edge of the ultrasonic vibrator to the inside thereof.

With this configuration, an electric short circuit or damage due to the contact between the horn-side connection terminal and the electrode of the ultrasonic vibrator can be more reliably avoided.

In accordance with another aspect of the invention, there is provided a skin care device having the ultrasonic generator above, the ultrasonic wave emitting surface of the flat plate-like horn forming a skin contact surface.

Furthermore, the present invention is directed to a skin care device comprising the ultrasonic generator configured as above, wherein the ultrasonic wave emitting surface of the flat plate-like horn forms a skin contact surface. With this configuration, a small-sized, lightweight, cost-effective skin care device can be provided. Further, in the skin care device, the electric power is reliably supplied to the ultrasonic vibrator through the flat plate-like horn.

In accordance with the present invention, it is possible to reduce the size, weight and cost of a device by employing a flat plate-like horn. Moreover, the present invention is capable of providing an ultrasonic generator that can reliably supply electric power to the ultra sonic vibrator through the horn and a skin care device using the ultrasonic generator.

The objects and features of the present invention will become apparent from the following description of embodiment given in conjunction with the accompanying drawings, in which:
Fig. 1 is a side cross sectional view showing the whole view of a skin care device in accordance with an embodiment of the present invention;
Figs. 2A and 2B are front and rear views illustrating the whole view of the skin care device;
Fig. 3 is an exploded perspective view showing major parts of the skin care device;
Fig. 4 is a side cross sectional view showing the major parts of the skin care device;
Fig. 5 is a plan view of the major parts of the skin care device seen from below;
Figs. 6A and 6B are explanatory views illustrating the spring configuration of the skin care device, in which Fig. 6A shows a vibrator side connection terminal and Fig. 6B shows a horn side connection terminal;
Figs. 7A to 7C are another explanatory views illustrating the spring configuration of the skin care device, in which Fig. 7A shows a coil spring configuration, Fig. 7B shows another coil spring configuration, and Fig. 7C shows a contact pin configuration;
Fig. 8 is an explanatory view illustrating an amplitude of an ultrasonic vibrator of the skin care device;
Fig. 9 is an explanatory view illustrating a case of an ultrasonic vibrator of the skin care device in which a restriction member is not prepared;
Fig. 10 is an explanatory view illustrating a modified electrode of the ultrasonic vibrator of the skin care device; and
Fig. 11 is a side cross sectional view showing major parts of a conventional skin care device.

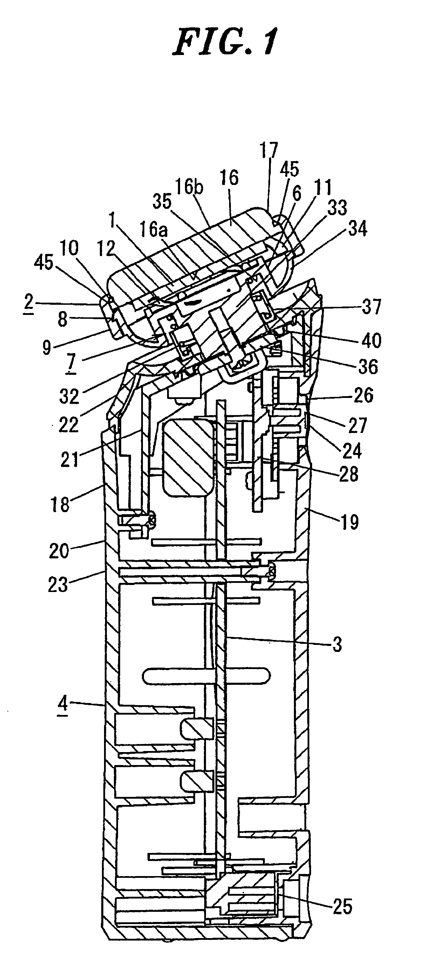

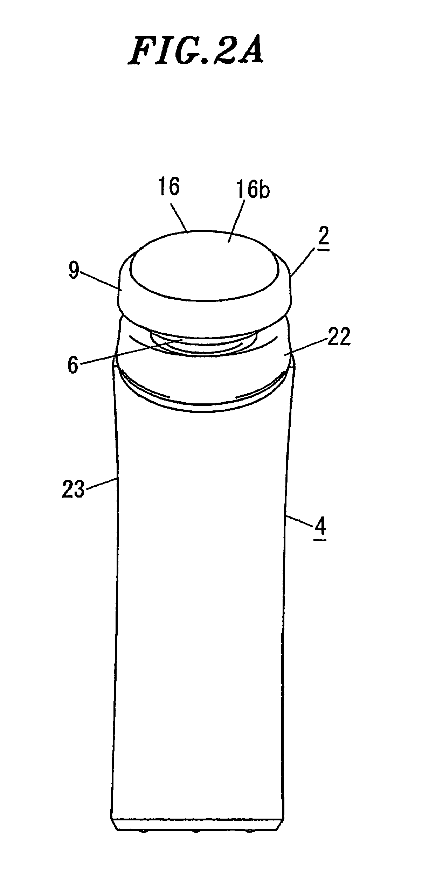

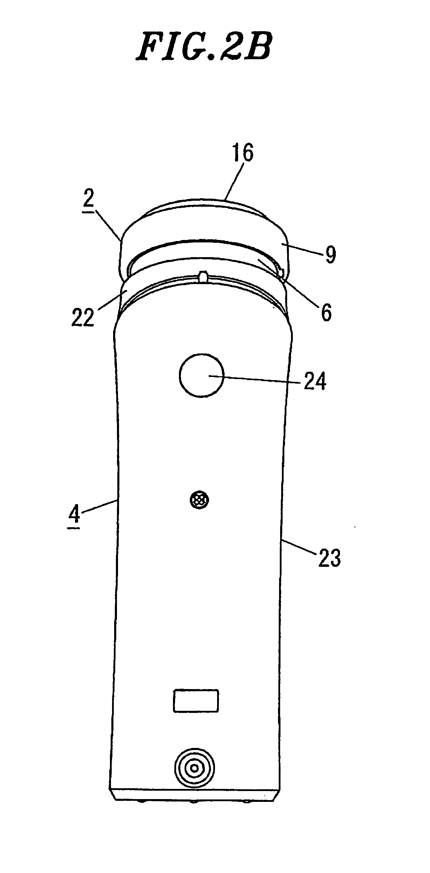

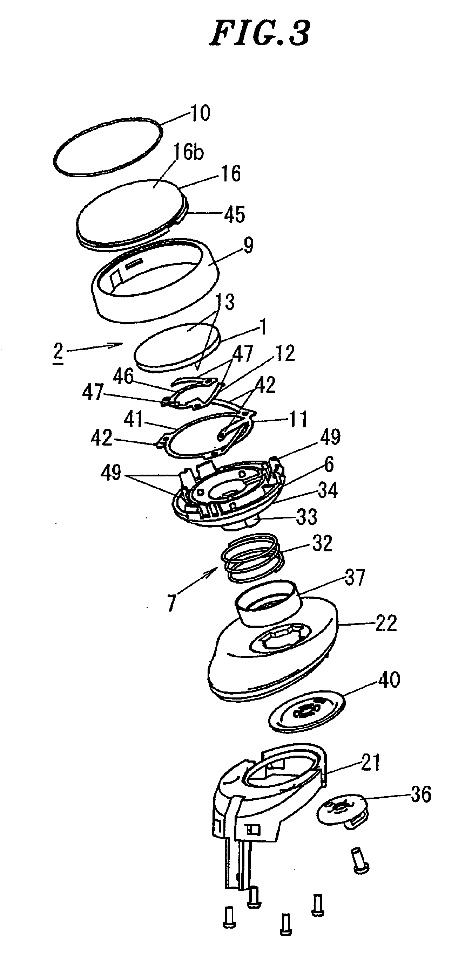

Embodiments of the present invention will now be described with reference to accompanying drawings which form a part hereof. Figs. 1, 2A and 2B show the whole view of a skin care device in accordance with an embodiment of the present invention. Fig. 3 shows main parts of the skin care device. The skin care device in accordance with the present embodiment includes an ultrasonic generator and is designed to provide an aesthetic effect by transmitting the ultrasonic vibration emitted from an ultrasonic wave emitting surface 16b of the ultrasonic generator to a skin surface.

The skin care device in accordance with the present embodiment (i.e., the ultrasonic generator forming a part of the skin care device) includes, as its major components, a head block 2 with a built-in ultrasonic vibrator 1 and a grip block 4 having a built-in control circuit 3 that applies an alternating electric field to the ultrasonic vibrator 1 to generate ultrasonic vibration.

First, description will be made on the basic configuration of the head block 2. The head block 2 includes a horn 16 of disc shape (hereinafter referred to as a "flat plate-like horn 16") made of metal such as aluminum or the like, an ultrasonic vibrator 1 adhesively fixed to one flat surface of the flat plate-like horn 16, a base portion 6 forming the bottom portion of the head block 2, and a water-proof cover 9.

The water-proof cover 9 engages with a flange-like support 45 projecting from the outer circumferential surface 17 of the flat plate-like horn 16 and, at the same time, engages with a hook-like engagement part 8 protruding from the outer surface of the base portion 6. By attaching and fixing the flat plate-like horn 16 to the base portion 6 through the water-proof cover 9, the flat surface (hereinafter called an "ultrasonic wave emitting surface 16b") of the flat plate-like horn 16, the one opposite to the flat surface (hereinafter called a "connection surface 16a") to which the ultrasonic vibrator 1 is fixed, can be exposed to the outside such that it protrudes obliquely and upwardly as viewed in the figure.

An 0-ring 10 is interposed between the support portion 45 of the flat plate-like horn 16 and the water-proof cover 9.

Also provided within the head block 2 are a vibrator-side connection terminal 12 and a horn-side connection terminal 11 for supplying electricity to the ultrasonic vibrator 1. The horn-side connection terminal 11 makes elastic contact with one of a pair of electrodes 13 (see Fig. 3) provided in the ultrasonic vibrator 1, so that the horn-side connection terminal 11 is directly and electrically connected to the corresponding electrode 13.

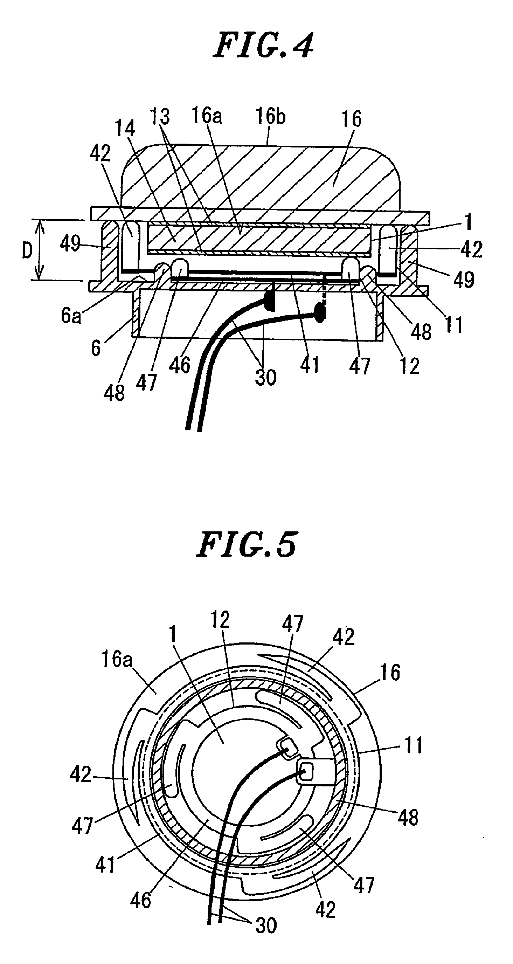

Furthermore, the vibrator-side connection terminal 12 makes elastic contact with the connection surface 16a of the flat plate-like horn 16, so that the vibrator-side connection terminal 12 is electrically connected to the other electrode 13 of the ultrasonic vibrator 1. The connection terminals 11 and 12 are both fixed to the base portion 6 and electrically connected to the control circuit 3 of the grip block 4 via lead lines 30 (see Fig. 4).

Next, description will be made on the basic configuration of the grip block 4. The grip block 4 includes a bottom-closed tubular main housing 20 having a front housing 18 and a rear housing 19 screw-fixed thereto, a generally ring-shaped support base 21 screw-fixed to the opening of the main housing 20 and a top cover 22. The main housing 20, the support base 21 and the top cover 22 form an overall housing 23 of the grip block 4.

Received within the housing 23 are the control circuit 3, an operation switch 24 and a power source jack 25. The operation switch 24 includes a switch cover 26 attached to the rear housing portion 19, a switch boss 27 and a switch substrate 28. The switch substrate 28 and the power source jack 25 are electrically connected to the control circuit 3 via lead lines (not shown).

Next, description will be made on a support mechanism 7 for movably supporting the head block 2 on the grip block 4. In the present embodiment, the support mechanism 7 includes a coil spring 32 as an elastic member interposed in a compressed state between the base portion 6 forming the bottom portion of the head block 2 and the top cover 22 forming the top portion of the grip block 4. The bottom of the base portion 6 includes a columnar central penetration portion 33, a peripheral edge portion 34 surrounding the penetration portion 33, and an annular groove portion 35 formed between the penetration portion 33 and the peripheral edge portion 34.

The penetration portion 33 is inserted into the central aperture of the top cover 22. A disc-like stopper 36 is screw-fixed to the tip end of the penetration portion 33 within the top cover 22. The stopper 36 is a member that prevents the head block 2 from slipping through by making contact with the inner surface of the top cover 22 through a water-proof cover 40 which will be described below.

A cylindrical water-proof member 37 is provided around the outer circumference of the coil spring 32 to surround the same. The coil spring 32 is pressed against the top cover 22 through the water-proof member 37. A sheet-like water-proof cover 40 is arranged inside the top cover 22 of the grip block 4. The water-proof member 37 and the water-proof cover 40 ensures that the liquid such as gel or the like used as an ultrasonic wave transmitting medium for skin care is prevented from infiltrating into the grip block 4, thereby avoiding circuit breakage or other troubles in a reliable manner.

Furthermore, the O-ring 10 arranged between the flat plate-like horn 16 and the water-proof cover 9 makes sure that the liquid such as gel or the like is prevented from infiltrating into the head block 2.

In the skin care device configured as above, if the operation switch 24 is turned on, an electric vibration generated by an alternating electric field applied through the connection terminals 11 and 12 is applied to the ultrasonic vibrator 1 from the control circuit 3, wherein the ultrasonic vibrator 1 converts the electric vibration into a mechanical vibration to thereby generate ultrasonic vibration. Then, when one touches skin of a body with the ultrasonic wave emitting surface 16b of the flat plate-like horn 16 while gripping the grip block 4 with one hand, the ultrasonic vibration generated in the ultrasonic vibrator 1 is transmitted to a skin surface or other destinations of a living body, thus attaining a desired aesthetic effect.

Next, description will be made on the characterized parts of the present invention with reference to Figs. 4 to 10. As set forth earlier, in the skin care device in accordance with the present embodiment, the flat plate-like horn 16 of disc shape having the oppositely positioned flat surfaces, i.e., the connection surface 16a and the ultrasonic wave emitting surface 16b, is used as the horn for receiving the ultrasonic vibration from the adhesively fixed ultrasonic vibrator 1 and emitting the ultrasonic vibration toward a target point.

The ultrasonic vibrator 1 is fabricated by interposing a disc-like piezoelectric material 14 between a pair of electrodes 13 such that flat surfaces of both sides of the piezoelectric material 14 are disposed respective electrodes 13. One of the electrodes 13 of the ultrasonic vibrator 1 is adhesively fixed to the connection surface 16a of the flat plate-like horn 16 by using an electrically conductive adhesive (not shown).

The horn-side connection terminal 11 and the vibrator-side connection terminal 12 for supplying an electric power to the ultrasonic vibrator 1 are fixed on a surface (refer to as a terminal holding surface) 6a of the base portion 6 facing the 16a of the flat plate-like horn 16, in which the horn-side connection terminal 11 surrounds the whole vibrator-side connection terminal 12.

The horn-side connection terminal 11 will now be described. As shown in Figs. 3 to 5, the horn-side connection terminal 11 is a plate spring member having a connection portion 41 of ring shape and a plurality of (three in this embodiment) elastic pieces 42 of arm shape each protruded from an outer circumference of the connection portion 41 with same interval and extending along the circumference.

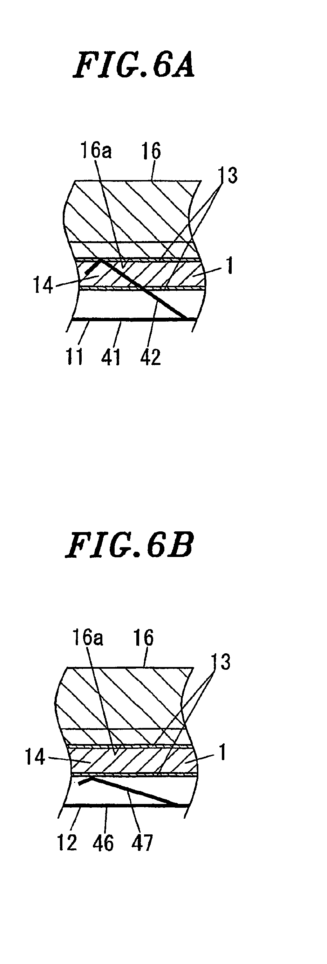

The connection portion 41 is fixed to the terminal holding surface 6a of the base portion 6 and connected to a lead wire 30. The elastic pieces 42 are installed in a state that the elastic pieces 42 are bent from the connection portion 41 toward flat plate-like horn 16, thereby making leading ends of the elastic pieces 42 elastically contact with the flat plate-like horn 16 (refer to Fig. 6A).

The circumferential portion of the one side plate 16a of the flat plate-like horn 16 surrounding the ultrasonic vibrator 1 is the area being contacted by each elastic piece 42. Since the connection surface 16a of the flat plate-like horn 16 is larger than the flat plate of the ultrasonic vibrator 1, there is a region in the connection surface 16a for the contact with the horn-side connection terminal 11 surrounding the ultrasonic vibrator 1 which is centrally contacted thereto.

The region which is not contacted by the ultrasonic vibrator 1 has a width, preferably, in a range from 1mm to 5mm from the circumferential edge of the connection surface 16a. Further, it is preferable that the leading end of each of the elastic pieces 42 of the horn-side connection terminal 11 is elastically contacted to the connection surface 16a at a portion more than 0.05mm away from the ultrasonic vibrator 1.

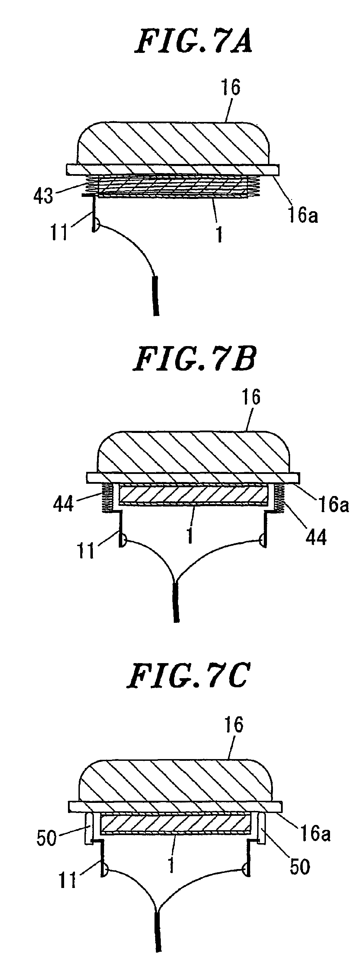

In this example, although the horn-side connection terminal 11 is a form of the plate spring member, other type of spring as shown in Figs. 7A to 7C may form the horn-side connection terminal 11. A modified example as shown in Fig. 7A forms the horn-side connection terminal 11 by a single coil spring 43 surrounding the whole ultrasonic vibrator 1; another modified example as shown in Fig. 7B forms the horn-side connection terminal 11 by a plurality of coil springs 44 disposed at the outside of the ultrasonic vibrator 1; and still another modified example as shown in Fig. 7C forms the horn-side connection terminal 11 by a plurality of contact pins disposed at the outside of the ultrasonic vibrator 1. The springs in either case may be made of a light alloy, preferably, a copper alloy.

As shown in Fig. 5, the horn-side connection terminal 11 is patterned after the form of the ultrasonic vibrator 1 contacted to the flat plate-like horn 16. Specifically, each elastic piece 42 of the horn-side connection terminal 11 to be contacted to the connection surface 16a at the peripheral portion thereof has an arc shape conforming to the peripheral edge of the ultrasonic vibrator 1 of circular shape. Accordingly, the horn-side connection terminal 11 is disposed at the connection surface 16a without contacting the ultrasonic vibrator 1 while efficiently using the small area.

Further, each of the elastic pieces 42, especially the leading end portion thereof, has an acute shape which becomes further distanced away from the ultrasonic vibrator 1 as the leading end portion approaches closer to the leading end contacting the connection surface 16a. Meanwhile, the horn-side connection terminal 11 is formed by cutting the whole connection portion 41 and the elastic pieces 42 from a flat plate spring and bending the elastic pieces 42 at the base thereof toward the flat plate-like horn 16.

Accordingly, it is possible that each of the end portions of the elastic pieces 42 is bent toward ultrasonic vibrator 1 depending on the way of a worker bending the corresponding one of the elastic pieces 42. Even in this case, however, the elastic pieces 42 of the acute shape are prevented from contacting the ultrasonic vibrator 1, which might cause a short circuit or damage.

Next, the vibrator-side connection terminal 12 will now be described. As shown in Figs. 3 to 5, the vibrator-side connection terminal 12 is smaller than the horn-side connection terminal 11 and is generally of a similar shape with the horn-side connection terminal 11. Specifically, the vibrator-side connection terminal 12 has a configuration similar to the horn-side connection terminal 11 in which the vibrator-side connection terminal 12 is a plate spring member having a connection portion 46 of ring shape and a plurality of (three in this embodiment) elastic pieces 47 of arm shape each protruded from an outer circumference of the connection portion 46 with same interval and extending along the circumference. The connection portion 46 is fixed to the terminal holding surface of the base portion 6 and connected to the lead wire 30.

The elastic pieces 47 are installed in a state that the elastic pieces 47 are bent from the connection portion 46 toward the ultrasonic vibrator 1, thereby making leading ends of the elastic pieces 47 elastically contact with ultrasonic vibrator 1 (refer to Fig. 6B).

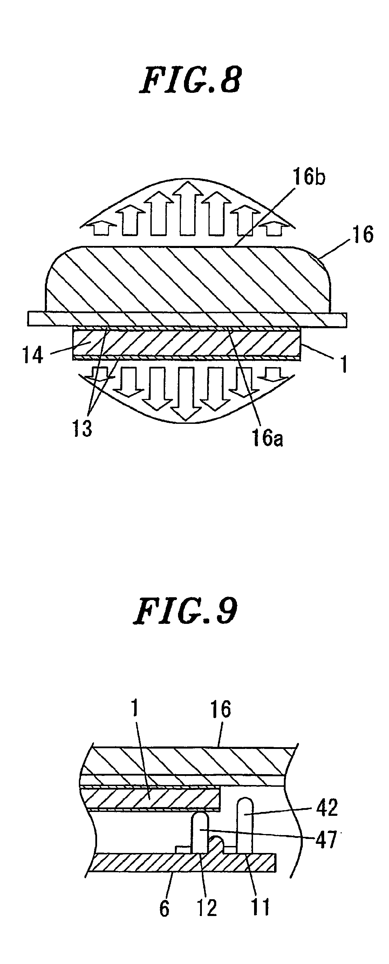

Each elastic piece 47 of the vibrator-side connection terminal 12 is preferred to make a contact with the electrode 13 of the ultrasonic vibrator 1 at an area that vibrates least in the circumferential surface of the electrode 13 to prevent any defects at the contact as much as possible. The ultrasonic vibrator 1 in this example has a disk shape and has an amplitude profile of vibration of a convex shape as schematically shown in Fig. 8 so that it shows a largest amplitude at a center of the disk while the amplitude is getting smaller as one proceeds to the circumferential edge of the ultrasonic vibrator 1. Accordingly, the leading end of the elastic piece 47 is made to have contact with the ultrasonic vibrator 1 at a circumferential portion of the ultrasonic vibrator 1 having a smaller amplitude of the vibration than other area.

Further, as described above, the horn-side connection terminal 11 is disposed at an outer portion of the terminal holding surface 6a of the base portion 6, the vibrator-side connection terminal 12 is disposed at an inner portion of the terminal holding surface 6a of the base portion 6, and an insulation member 48 is disposed between the horn-side connection terminal 11 and the vibrator-side connection terminal 12 to prevent them from contacting with each other. The insulation member 48 is a protection wall which is integrally formed with the base portion 6 and has a width of about 1mm of ring shape, thereby reliably preventing the horn-side connection terminal 11 and the vibrator-side connection terminal 12 from contacting with each other.

Furthermore, to more reliably prevent the horn-side connection terminal 11 and the vibrator-side connection terminal 12 from contacting with each other, it is preferable to make the direction of the circumferential portion of the horn-side connection terminal 11 and that of the vibrator-side connection terminal 12 go amiss from each other to ensure largest distances between the elastic pieces 42 and the elastic pieces 47 while making the number of the elastic pieces 42 of the horn-side connection terminal 11 and that of the elastic pieces 47 of the vibrator-side connection terminal 12 same as in the example above.

Restriction members 49 maintaining a distance between the terminal holding surface 6a of the base portion 6 and the connection surface 16a of the flat plate-like horn 16 by a predetermined distance D are disposed at outer places of the horn-side connection terminal 11 in the terminal holding surface 6a of the base portion 6 (refer to Figs. 3 and 4). The restriction members 49 are a columnar protrusions integrally formed with the base portion 6. During installation, the connection surface 16a of the flat plate-like horn 16 approaches the leading edges of the restriction members 49 to contact with them, thereby maintaining the predetermined distance D.

The predetermined distance D is set to maintain appropriate contact pressures when the elastic pieces 42 of the horn-side connection terminal 11 make contacts with the flat plate-like horn 16 and the elastic pieces 47 of the vibrator-side connection terminal 12 contact with the ultrasonic vibrator 1, respectively.

Optimal contact pressures at the elastic pieces 42 of the horn-side connection terminal 11 and those at the elastic pieces 47 of the vibrator-side connection terminal 12 are about in a range from 0.05 to 0.2 kgf. However, in case the predetermined distance D cannot be insured, it is difficult to maintain the optimal contact pressures. For example, when the distance between the terminal holding surface 6a and the connection surface 16a is larger than the predetermined distance D without the restriction members 49, it is possible that the horn-side connection terminal 11 may not make a contact with the flat plate-like horn 16 as shown in Fig. 9.

Referring to Fig. 10, another modified example of the ultrasonic vibrator 1 is described. In the modified ultrasonic vibrator 1, the electrode 13 electrically contacting with the vibrator-side connection terminal 12 among the two electrodes 13 is formed on the ultrasonic vibrator 1 while axially removing a cutout portion d from the circumferential edge of the ultrasonic vibrator 1 to the inside thereof.

The cutout portion d is employed to reliably prevent the elastic pieces 42 of the horn-side connection terminal 11 and the electrode 13 of the ultrasonic vibrator 1 from contacting with each other in case when the ultrasonic vibrator 1 is fixed at a position misaligned with a center of the flat plate-like horn 16, or when the elastic pieces 42 of the horn-side connection terminal 11 are additionally bent inward, and so forth. Therefore, a short circuit or damage can be reliably avoided.

The cutout portion d may be provided at whole circumferential portion of the electrode 13, or may be provided at near the elastic pieces 42 of the horn-side connection terminal 11. Since the cutout portion d is formed at whole circumferential portion of the electrode 13 in another modified example shown in the Fig. 10, an edge of the electrode 13 has a circular shape conforming to an edge of the ultrasonic vibrator 1. In this case, it is preferable that the edge of the electrode 13 of circular shape has a diameter smaller than that of the edge of the ultrasonic vibrator 1 of circular shape by about 5 to 10 %.

The ultrasonic generator configured as above may be applied to a variety of devices other than the skin care device. Devices to which the present invention is applicable are not limited to the one for beauty care but may include the ones for performing various kinds of ultrasonic processing such as diagnosis, machining, bonding, cleansing, agent permeation acceleration, diffusion, dispersion, emulsification and atomization. The same effects as described above can be attained in these cases.

While the invention has been shown and described with respect to the embodiment, it will be understood by those skilled in the art that various changes and modifications may be made without departing from the scope of the invention as defined in the following claims.

## Claims

1. An ultrasonic generator comprising:
an ultrasonic vibrator generating ultrasonic vibration; and
a horn having a connection surface connected to the ultrasonic vibrator and an ultrasonic wave emitting surface from which ultrasonic waves are emitted toward a target point,
wherein the horn includes a flat plate-like horn having opposite flat surfaces, one of which forms the connection surface and the other of which forms the ultrasonic wave emitting surface, and wherein a horn-side connection terminal contacts a circumferential portion of the connection surface surrounding the ultrasonic vibrator at a plurality of place thereof to electrically connect to one electrode of the ultrasonic vibrator through flat plate-like horn.

2. The ultrasonic generator of claim 1, wherein the horn-side connection terminal elastically contacts the flat plate-like horn by using a spring structure.

3. The ultrasonic generator of claim 1 or 2, wherein the horn-side connection terminal is of a shape conforming to an edge of the ultrasonic vibrator connected to the flat plate-like horn.

4. The ultrasonic generator of claim 2 or 3, wherein the horn-side connection terminal includes elastic pieces of an arm shape elastically contacting the flat plate-like horn, the leading end portions of the elastic pieces becoming gradually distanced away from the ultrasonic vibrator as the leading end portions go to the leading ends of the elastic pieces and being bent toward the flat plate-like horn.

5. The ultrasonic generator of any one of claims 1 to 4, wherein a vibrator-side connection terminal contacts a circumferential portion of the other electrode of the ultrasonic vibrator surrounded by the vibrator-side connection terminal at a plurality of place thereof for an electric connection.

6. The ultrasonic generator of claim 5, wherein the vibrator-side connection terminal contacts a portion of the ultrasonic vibrator where the ultrasonic vibration is least.

7. The ultrasonic generator of claim 5 or claim 6, wherein an insulation member is disposed between the outer horn-side connection terminal and the inner vibrator-side connection terminal.

8. The ultrasonic generator of one of claims 5 to 7, further comprising:
a base portion mounting the horn-side connection terminal and the vibrator-side connection terminal thereon, each having a spring structure; and
a restriction member maintaining a predetermined distance between the base portion and the flat plate-like horn to maintain predetermined contact pressures between the horn-side connection terminal and the flat plate-like horn and between the vibrator-side connection terminal and the ultrasonic vibrator, respectively.

9. The ultrasonic generator of one of claims 5 to 8, wherein an electrode contacting with the vibrator-side connection terminal among both electrodes has a cutout portion d which is formed from the circumferential edge of the ultrasonic vibrator to the inside thereof.

10. A skin care device comprising the ultrasonic generator of any one of claims 1 to 9, wherein the ultrasonic wave emitting surface of the flat plate-like horn forms a skin contact surface.
